# EUROPEAN PATENT SPECIFICATION

(11) **EP 3 599 014 B1**
(45) Date of publication and mention of the grant of the patent: **23.12.2020**
(21) Application number: 18185807.7
(22) Date of filing: 26.07.2018
(51) Int. Cl.: B01J 8/18, B01J 8/26, B01J 8/02, C07C 9/04, C01B 3/00, B01J 19/24

(54) **METHANE SYNTHESIS SYSTEM**
METHANSYNTHESESYSTEM
SYSTÈME DE SYNTHÈSE DE MÉTHANE

(43) Date of publication of application: 29.01.2020
(60) Divisional application: 20161246.2
(73) Proprietor: Doosan Lentjes GmbH, 40880 Ratingen (DE)
(72) Inventor: KEHRMANN, Kai Matthias, 44577 Castrop-Rauxel (DE); EINICKE, Clemens, 40699 Erkrath (DE); NARIN, Oguzhan, 45549 Sprockhövel (DE); BROSCH, Björn, 45136 Essen (DE); STETTER, Thomas, 40545 Düsseldorf (DE)
(74) Representative: Feucker, Max Martin

(56) References cited:
- US-A- 2 489 347
- US-A- 2 842 102
- US-A- 3 360 867
- Oxana A. Kholdeeva: "SELECTIVE OXIDATION OF AROMATIC RINGS" In: "Arene Chemistry: Reaction Mechanisms and Methods for Aromatic Compounds, First Edition. Edited", 25 November 2015 (2015-11-25), Wiley Online Library, XP055586329, pages 365-398, DOI: 10.1002/9781118754887.ch14, * page 385 *

## Description

The present invention relates to a methane synthesis system, which comprises one common supply line at least for carbon oxide (such as carbon monoxide CO or carbon dioxide CO₂) and hydrogen (H₂), a bed reactor connected to the one supply line and a common discharge line.

In the prior art methane synthesis systems are known with one single packed bed reactor. In such packed bed reactors the catalysts (such as nickel or ruthenium) for supporting the reaction of carbon oxide and hydrogen to hydrocarbons (i.e. methane and/or methanol) are stationary fixed within the reactor. As the reaction is exothermic the produced heat needs to be extracted from the packed bed reactor, which is usually achieved in multiple steps. The known systems are usually adapted for a constant supply of hydrogen and carbon oxides, which makes the known methane synthesis inflexible, if the supply of hydrogen and carbon oxides changes.

In particular, when the hydrogen supplied to methane synthesis systems is to be produced by electrolysis using an electricity surplus of renewable energy sources, the methane synthesis systems need to be operated in a more flexible way. In such a configuration there may be no hydrogen supply for a certain time, whereas the hydrogen supply might start quickly to different levels. Accordingly, also a methane synthesis system needs to be provided which can run up quickly in an energy saving manner, when the supply of hydrogen and carbon oxides starts.

US 2,489,347 discloses a method for the oxidation of aromatic hydrocarbons. US 3,360,867 discloses a system and a method for treating solid materials which shall by dried or moistened. US 2,842,102 discloses a steam generation plant.

Accordingly, it is an object of the invention to avoid the drawbacks of the prior art and to provide a methane synthesis system, with which a methane synthesis system can be operated more flexible, when the supply of hydrogen changes and in particular when the supply of hydrogen and carbon oxides starts.

The object is achieved by a methane synthesis system with the features of the independent claim. Advantageous embodiments of the system are described in the subclaims and in the description, wherein single features of the advantageous embodiments can be combined with each other in a technical meaningful manner.

The object is in particular achieved with a system with the above described features, wherein the system comprises at least one second bed reactor selectively connectable to the one supply line.

The basic idea behind this solution is to provide one or more additional bed reactors which can be connected selectively to the one common supply line when the supply of carbon oxides and hydrogen increases and which can be disconnected selectively from the one common supply line when the supply of carbon oxides and hydrogen decreases. This way the system can be easily adapted to different supply conditions of CO₂ and H₂. It is important that the system has only one single common supply line, through which a mixture of at least carbon oxides and hydrogen can be provided. The provided gas stream may contain further components in addition to the carbon oxides (such as carbon monoxide CO and/or carbon dioxide CO₂) and hydrogen (H₂).

The first and at least the one second bed reactor may be embodied as packed bed reactor or as fluidized bed reactor. Preferably, all (the first and all second) bed reactors are embodied as fluidized bed reactors. In a fluidized bed reactor the catalysts (such as for example nickel and ruthenium) for enhancing the reaction of carbon oxides and H₂ to hydrocarbons (such as methane (CH₄) and/or methanol (CH₃OH)) are provided by solid particles (particulate matter) in the reactor, wherein the gas stream comprising carbon oxides and H₂ is introduced at the bottom, so that the solid particles and the gas behave as a fluid. Fluidized bed reactors as such are known from the prior art. By the so called fluidization of the solid particles the contact between the particles and the gas is greatly enhanced, whereby the reaction and heat transfer is enhanced. The solid particles (comprising catalysts) are usually provided as loose filling within the fluidized bed reactor. Depending on the velocity of the provided gas the fluidized bed can be embodied as stationary, bubbling or circulating fluidized bed. Fluidized bed reactors usually have a fluidized bottom which has at least one nozzle for providing the gas stream and/or a gas distribution plate above the gas supply.

In order that the reaction of CO₂ and H₂ begins, a temperature of about 200° Celsius is to be provided. After the reaction has started no additional energy needs to be provided to the respective bed reactor. Accordingly, it is suggested that at least one heater is provided at least for the first bed reactor. The heater is arranged in such a way that the gas stream (comprising H₂ and CO/CO₂) to one or all bed reactors can be heated or that the packed bed or the fluidized bed in the bed reactor can be directly heated.

In one embodiment one single heater is provided, which is arranged relative to the one common supply line ahead of the first or ahead of all bed reactors such that the gas stream to the first bed reactor or the whole provided gas stream can be heated. By providing a heater in/at the common supply line only one heater is necessary to promote the catalytic reaction in all (first and second) bed reactors.

In a further embodiment, a heater can be provided for each bed reactor, so that all bed reactors are provided with a heater and can be heated separately.

In an even further embodiment one single heater is provided only for the first bed reactor. By providing only one heater for the first (fluidized) bed reactor it is possible to promote the catalytic reaction in the first bed reactor only, while with the heat of the exothermic catalytic reaction the further bed reactor(s) are heated, such that the catalytic reaction in the further (second) bed reactor can be promoted without an additional heater. Accordingly, the dimension of the heater for only a first bed reactor can be lower.

When a heater is provided for a specific bed reactor, preferably the heater is arranged at (near) the inlet to the respective bed reactor, so that the gas stream provided to the respective bed reactor from the common supply line can be heated selectively.

Preferably the at least one heater is an electrical heater.

In order that the heat of one bed reactor is transferred to a second bed reactor, the first bed reactor and at least one second bed reactor have a common wall. Preferably, two or more second bed reactors each have a common wall with the first bed reactor. This way heat energy from the first bed reactor can be transferred to multiple second bed reactors. Each common wall may be water cooled.

For starting the operation of the methane synthesis system the first bed reactor can be connected to the common supply line, such that CO/CO₂ and H₂ are only supplied to the first bed reactor, wherein the gas supplied to the first bed reactor is heated, in order to promote the catalytic reaction within the first bed reactor. After the catalytic reaction has started in the first bed reactor and/or when a H₂/CO₂ volume is provided through the common supply line, which exceeds the capacity of the first reactor, at least one further (second) bed reactor can be (selectively) connected to the supply line, so that CO₂ and H₂ is supplied to the second bed reactor(s), wherein the thermal energy for the starting temperature of the catalytic reaction within the second bed reactor(s) can be supplied by the already operated (first) bed reactor(s). This way the system can not only be adapted to different supplies of CO/CO₂ and H₂ easily, but also energy can be saved by using the heat generated in the already operated (first) bed reactor(s) to promote the starting of the reaction in a further bed reactor.

In particular, when the bed reactors are embodied as fluidized bed reactors a heat exchanger is arranged at least in the first bed reactor. Preferably a heat exchanger is arranged in every bed reactor. By combining a fluidized bed with a heat exchanger, the heat exchange between the fluidized bed and the heat exchanger is highly efficient. Such a heat exchanger may extend from an outer wall of the bed reactor into the fluidized bed. The heat exchanger can for example be embodied by a tube, which extends from a wall into the bed reactor and which is preferably formed at least partly helically or meandering. Each heat exchange tube may have an inlet and an outlet for heat exchange medium (i.e. water), wherein the volume flow of the heat exchange medium through the heat exchange tube might be controllable.

At the beginning of the operation of the synthesis system the heat exchanger of the first bed reactor may be used as heater for the first bed reactor. In this case the heat exchange medium may be heated before being supplied through the heat exchange tube into the first bed reactor, thereby heating the first bed reactor to the critical temperature for the catalytic reaction.

It may also be possible that the heat exchanger of the first bed reactor can be (selectively) connected to the heat exchanger(s) of the second bed reactor(s), such that the heat generated in the first bed reactor can be transferred to the second bed reactor(s) in order to promote the catalytic reaction in the second bed reactor(s), when the second bed reactor(s) are run up. In a stationary mode of operation of the system the heat withdrawn by the heat exchanger(s) and eventually by the water cooled wall(s) can be used further.

In order to selectively connect the first and/or second bed reactor(s) to the one common supply line a setting configuration might be embodied, with which the bed reactors can be connected and disconnected selectively from the supply line. For example, the setting configuration may comprise one or more setting element(s) with which the gas flow to one or more bed reactors can be interrupted or enabled. For example, the setting elements may be embodied as a flap, with which the flow through a pipe can be interrupted. The setting configuration may be connected to a control unit with which the status of the setting elements can be controlled and/or altered.

In one embodiment, each bed reactor can be connected and disconnected separately from the supply line by a separate setting element.

In a further embodiment, a group of bed reactors can be connected and disconnected from the common supply line by a single setting element.

In order to reuse particles and entrains from the fluidized bed, a separator for particulate matter (solid particles, i.e. catalytic material) may be embodied. For example, a separator for particulate matter may be embodied in each fluidized bed reactor. Alternatively or additionally a common separator for particulate matter may be connected to the common discharge line. The separator may be embodied as filter, cyclone separator or magnetic separator.

In an embodiment, a recirculation line connects the common discharge line to the common supply line, so that gases discharged from the multiple bed reactors may be recirculated into the common supply line.

Preferably, the system is embodied as a compact unit having a frame for supporting the multiple bed reactors, wherein exactly one connection to the one supply line is provided. Preferably, the common supply line of the methane synthesis system is connected to an electrolysis unit for water as source for hydrogen, while the electrolysis unit is operated with electrical energy from renewable energy sources.

The system comprises preferably at least two second bed reactors and more preferably at least four second bed reactors.

The first bed reactor and the second bed reactor are usually configured the same. The bed reactor which is coupled to the common supply line at the beginning of the operation of the system is usually considered the first bed reactor. But it may also be possible that more than one bed reactor is connected to the supply line at the beginning of the operation of the system. It may also be possible, that by respective settings of the setting system a different bed reactor is connected to the supply line at each start of the operation. Accordingly, it is desired, that at least two bed reactors are embodied, from which at least one is selectively connectable and disconnectable from the supply line.

The invention and the technical background is described in the following with regard to the figures which show embodiments of the invention. The figures show schematically
- Figure 1:: a first embodiment of a methane synthesis system in cross sectional view,
- Figure 2:: a second embodiment in top view and
- Figure 3:: a third embodiment in side view.

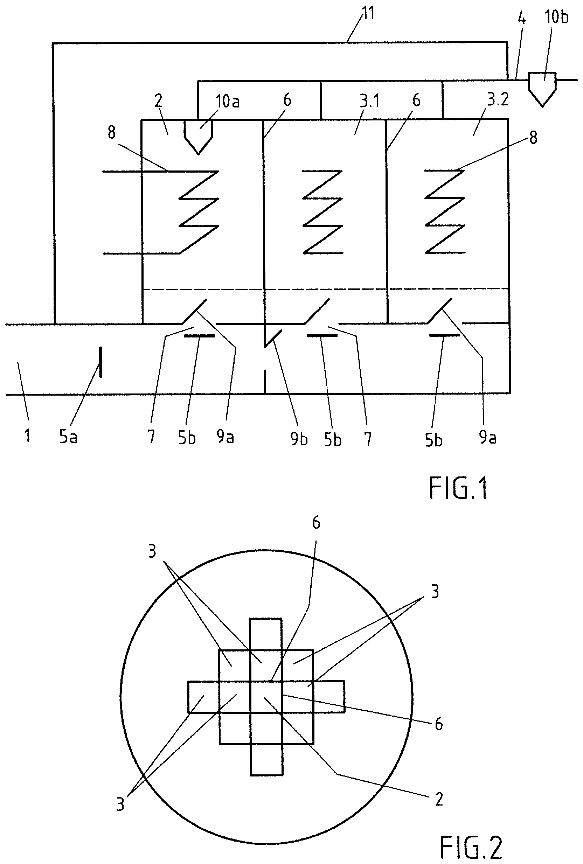

The methane synthesis system shown in figure 1 comprises a first bed reactor 2 and multiple second bed reactors 3 of which only two second bed reactors 3.1, 3.2 are shown. Adjacent bed reactors are separated by common water cooled walls 6. The bed reactors 2, 3.1, 3.2 are embodied as fluidized bed reactors comprising a loose filling of particulate matter being made of or being coated with catalytic material. The first bed reactor 2 and the second bed reactor 3.1, 3.2 can each be selectively connected to a common supply line 1 by setting elements 9. The setting elements 9a are embodied as flaps, with which each bed reactor 2, 3.1, 3.2 can be disconnected from the common supply line 1. With the setting element 9b a group of second bed reactors 3.1, 3.2 can be connected and disconnected to the common supply line 1.

The system shown in figure 1 comprises multiple heaters 5, which are embodied as electrical heaters. Electrical heater 5a is embodied to heat the whole gas stream provided through the supply line 1, whereas electrical heaters 5b are embodied to heat the gas stream which is provided through inlets 7 to the respective bed reactor 2, 3.1, 3.2.

Each bed reactor 2, 3 comprises a heat exchanger 8.

Ahead of the outlet of the first bed reactor 2 a separator 10a is embodied. A common separator 10b is embodied in the discharge line. Particulate matter can be separated from the gas stream with the separators 10a and 10b..

The discharge line 4 is connected by recirculation line 11 to the supply line 1.

In operation, a gas stream comprising carbon oxides (such as CO₂) and hydrogen (H₂) is supplied through the common supply line 1. At the beginning of the operation of the system the setting element 9b might be closed and the setting element 9a of the first bed reactor 2 may be opened, so that the gas stream is provided to the first bed reactor 2 only. With the aid of the heaters 5a, 5b the gas stream provided to the first bed reactor 2 might be heated above a critical temperature to start the catalytic reaction within the first bed reactor 2. After the catalytic reaction has started in the first bed reactor 2 and/or when a greater volume flow of gas is provided, the setting element 9b might be opened so that the gas can also be supplied to the second bed reactors 3.1, 3.2.

If the thermal energy provided by the exothermic reaction in the first bed reactor 2 is sufficient to heat the second bed reactor 3 above a critical temperature, no additional heat energy needs to be provided. If additional heat energy is needed, the gas supplied to the second bed reactor 3.1, 3.2 can be heated by the respective heaters 5b.

During a steady operation, the heat produced by the catalytic reaction of H₂ and CO/CO₂ within the bed reactors 2, 3.1, 3.2 can be withdrawn by the heat exchangers 8. Also, heat can be withdrawn by water supplied to the common walls 6. In a not shown embodiment the heat withdrawn from the first bed reactor 2 by the heat exchanger 8 can be supplied to a heat exchanger 8 of a second bed reactor 3, in order to raise the temperature in the second bed reactor 3 above the critical temperature for the catalytic reaction.

The methane produced within each bed reactor 2, 3.1, 3.2 and eventually residuals of CO₂ and H₂ and/or other components are discharged to a common discharge line 4, wherein particles (catalysts) can be withholded by the separator 10a in the bed reactor 2 or by separator 10b in the discharge line 4.

Figure 2 shows a top view on a second embodiment of a methane synthesis system, wherein a first bed reactor 2 is centrally arranged and wherein second bed reactors 3 surround the first bed reactor 2, so that the first bed reactor 2 has a common wall 6 with four second bed reactors 3. Only the embodiment of Figure 2 falls under the scope of independent system claim.

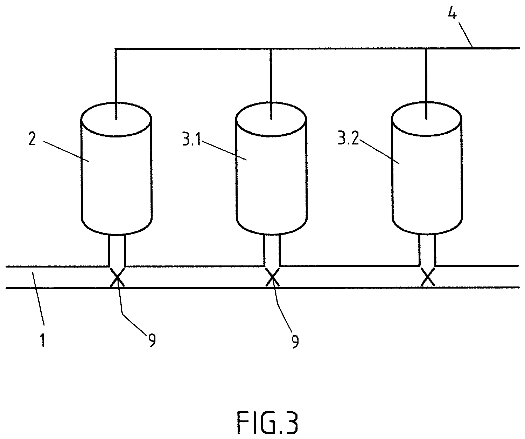

According to the further embodiment of figure 3, a first bed reactor 2 is provided, which is spatially separated from the second bed reactors 3.1, 3.2. Besides the spatial separation from each other the first bed reactor 2 and the second bed reactors 3.1, 3.2 might be embodied as the bed reactors shown in figure 1, for example each having a heater, a heat exchanger and a separator, while the cross sectional shape may differ.

### Reference numbers

- 1: supply line
- 2: first bed reactor
- 3: second bed reactor
- 4: discharge line
- 5: heater
- 6: common wall
- 7: inlet
- 8: heat exchanger
- 9: setting element
- 10: separator
- 11: recirculation line

## Claims

1. Methane synthesis system, comprising
- one common supply line (1) being connected at least to a carbon oxide source and to a hydrogen source,
- a first bed reactor (2) connected or selectively connectable to the one common supply line (1) and
- a common discharge line (4),
wherein the system comprises more than two second bed reactors (3) selectively connectable to the one common supply line (1), wherein the first bed reactor (2) and more than two second bed reactors (3) each have a common wall (6), wherein the first bed reactor (2) and the second bed reactors (3) comprise a catalyst for enhancing the reaction of carbon oxides and hydrogen to hydrocarbons.

2. System according to claim 1, wherein a heater (5) is provided at least for the first bed reactor (2).

3. System according to claim 1 or 2, wherein a heater (5) is provided for each bed reactor (2, 3).

4. System according to claim 2 or 3, wherein each heater is arranged at the inlet (7) to the respective bed reactor (2, 3).

5. System according to one of the preceding claims, wherein a heater (5) is provided in the one common supply line (1).

6. System according to one of the preceding claims, wherein the at least one common wall (6) is water cooled.

7. System according to one of the preceding claims, wherein a heat exchanger (8) is arranged at least in the first bed reactor (2).

8. System according to one of the preceding claims, wherein a setting configuration with at least one setting element (9) is embodied, with which gas flow from the one common supply line (1) to one or more bed reactor(s) (2, 3) can be interrupted or enabled selectively.

9. System according to claim 8, wherein a setting element (9) of the setting configuration is provided for each bead reactor (2, 3), so that the gas flow to each bed reactor (2, 3) from the one common supply line (1) can be interrupted or enabled separately.

10. System according to claim 8, wherein with a single setting element (9) of the setting configuration the gas flow to a group of bed reactors (3) from the one common supply line (1) can be interrupted or enabled.

11. System according to one of the preceding claims, wherein a separator (10) for particulate matter is embodied in each bed reactor (2, 3).

12. System according to one of the preceding claims, wherein a common separator for particulate matter is connected to the common discharge line (4).

13. System according to one of the preceding claims, wherein a recirculation line (11) connects the common discharge line (4) to the one common supply line (1).

14. System according to one of the preceding claims embodied as compact unit having a frame for supporting the multiple bed reactors (2, 3) and one connection for the one common supply line (1).

15. System according to one of the preceding claims, wherein all bed reactors (2, 3) are embodied as fluidized bed reactors.

16. Use of a methane system according to one of the preceding claims for synthesising methane

## Patentansprüche

1. Methansynthesesystem, umfassend
- eine gemeinsame Versorgungsleitung (1), die mindestens an eine Kohlenoxidquelle und an eine Wasserstoffquelle angeschlossen ist,
- einen ersten Schichtreaktor (2), der an die eine gemeinsame Versorgungsleitung (1) angeschlossen oder selektiv anschließbar ist, und
- eine gemeinsame Auslassleitung (4),
wobei das System mehr als zwei zweite Schichtreaktoren (3) umfasst, die selektiv an die eine gemeinsame Versorgungsleitung (1) anschließbar sind, wobei der erste Schichtreaktor (2) und mehr als zwei zweite Schichtreaktoren (3) jeweils eine gemeinsame Wand (6) aufweisen, wobei der erste Schichtreaktor (2) und die zweiten Schichtreaktoren (3) einen Katalysator zur Verstärkung der Reaktion von Kohlenoxiden und Wasserstoff zu Kohlenwasserstoffen umfassen.

2. System nach Anspruch 1, wobei eine Heizvorrichtung (5) mindestens für den ersten Schichtreaktor (2) vorgesehen ist.

3. System nach Anspruch 1 oder 2, wobei eine Heizvorrichtung (5) für jeden Schichtreaktor (2, 3) vorgesehen ist.

4. System nach Anspruch 2 oder 3, wobei jede Heizvorrichtung am Einlass (7) des jeweiligen Schichtreaktors (2, 3) angeordnet ist.

5. System nach einem der vorhergehenden Ansprüche, wobei eine Heizvorrichtung (5) in der einen gemeinsamen Versorgungsleitung (1) vorgesehen ist.

6. System nach einem der vorhergehenden Ansprüche, wobei die mindestens eine gemeinsame Wand (6) wassergekühlt ist.

7. System nach einem der vorhergehenden Ansprüche, wobei mindestens im ersten Schichtreaktor (2) ein Wärmetauscher (8) angeordnet ist.

8. System nach einem der vorhergehenden Ansprüche, wobei eine Einstellkonfiguration mit mindestens einem Einstellelement (9) ausgeführt ist, mit der der Gasstrom von der einen gemeinsamen Versorgungsleitung (1) zu einem oder mehreren Schichtreaktor(en) (2, 3) selektiv unterbrochen oder freigegeben werden kann.

9. System nach Anspruch 8, wobei ein Einstellelement (9) der Einstellkonfiguration für jeden Schichtreaktor (2, 3) vorgesehen ist, so dass der Gasstrom zu jedem Schichtreaktor (2, 3) von der einen gemeinsamen Versorgungsleitung (1) separat unterbrochen oder freigegeben werden kann.

10. System nach Anspruch 8, wobei mit einem einzigen Einstellelement (9) der Einstellkonfiguration der Gasstrom zu einer Gruppe von Schichtreaktoren (3) von der einen gemeinsamen Versorgungsleitung (1) unterbrochen oder freigegeben werden kann.

11. System nach einem der vorhergehenden Ansprüche, wobei ein Abscheider (10) für teilchenförmiges Material in jedem Schichtreaktor (2, 3) ausgeführt ist.

12. System nach einem der vorhergehenden Ansprüche, wobei ein gemeinsamer Abscheider für teilchenförmiges Material und die gemeinsame Auslassleitung (4) angeschlossen ist.

13. System nach einem der vorhergehenden Ansprüche, wobei eine Rücklaufleitung (11) die gemeinsame Auslassleitung (4) mit der einen gemeinsamen Versorgungsleitung (1) verbindet.

14. System nach einem der vorstehenden Ansprüche, das als kompakte Einheit ausgeführt ist, die eine Rahmen zum Tragen der Mehrschichtreaktoren (2, 3) und einen Anschluss für die eine gemeinsame Versorgungsleitung (1) aufweist.

15. System nach einem der vorhergehenden Ansprüche, wobei alle Schichtreaktoren (2, 3) als Wirbelschichtreaktoren ausgeführt sind.

16. Verwendung eines Methansystems nach einem der vorhergehenden Ansprüche zur Synthese von Methan.

## Revendications

1. Système de synthèse de méthane, comprenant
- une ligne d'alimentation commune (1) étant reliée à au moins une source d'oxyde de carbone et à une source d'hydrogène,
- un premier réacteur à lit (2) relié ou pouvant être relié de manière sélective à la ligne d'alimentation commune (1) et
- une ligne de décharge commune (4),
dans lequel le système comprend plus de deux seconds réacteurs à lit (3) pouvant être reliés de manière sélective à la ligne d'alimentation commune (1), dans lequel le premier réacteur à lit (2) et plus de deux seconds réacteurs à lit (3) possèdent chacun une paroi commune (6), dans lequel le premier réacteur à lit (2) et les seconds réacteurs à lit (3) comprennent un catalyseur permettant d'améliorer la réaction des oxydes de carbone et de l'hydrogène en hydrates de carbone.

2. Système selon la revendication 1, dans lequel un chauffage (5) est fourni pour au moins le premier réacteur à lit (2).

3. Système selon la revendication 1 ou la revendication 2, dans lequel un chauffage (5) est fourni pour chaque réacteur à lit (2, 3).

4. Système selon la revendication 2 ou la revendication 3, dans lequel chaque chauffage est agencé au niveau de l'entrée (7) de chaque réacteur à lit (2, 3) respectif.

5. Système selon l'une quelconque des revendications précédentes, dans lequel un chauffage (5) est fourni dans la ligne d'alimentation commune (1).

6. Système selon l'une quelconque des revendications précédentes, dans lequel l'au moins une paroi commune (6) est refroidie à l'eau.

7. Système selon l'une quelconque des revendications précédentes, dans lequel un échangeur thermique (8) est agencé dans au moins le premier réacteur à lit (2).

8. Système selon l'une quelconque des revendications précédentes, dans lequel une configuration de réglage avec au moins un élément de réglage (9) est incorporé, avec lequel un débit de gaz provenant de la ligne d'alimentation commune (1) vers l'un ou plusieurs réacteurs à lit (2, 3) peut être interrompu ou activé de manière sélective.

9. Système selon la revendication 8, dans lequel un élément de réglage (9) de la configuration de réglage est fourni pour chaque réacteur à lit (2, 3), de sorte que le débit de gaz vers chaque réacteur à lit (2,3) provenant de la ligne d'alimentation commune (1) peut être interrompu ou activé de manière séparée.

10. Système selon la revendication 8, dans lequel avec un unique élément de réglage (9) de la configuration de réglage, le débit de gaz vers un groupe de réacteurs à lit (3) provenant de la ligne d'alimentation commune (1) peut être interrompu ou activé.

11. Système selon l'une quelconque des revendications précédentes, dans lequel un séparateur (10) destiné à la matière particulaire est incorporé dans chaque réacteur à lit (2, 3).

12. Système selon l'une quelconque des revendications précédentes, dans lequel un séparateur commun destiné à la matière particulaire est relié à la ligne de décharge commune (4).

13. Système selon l'une quelconque des revendications précédentes, dans lequel une ligne de recirculation (11) relie la ligne de décharge commune (4) à la ligne d'alimentation commune (1).

14. Système selon l'une quelconque des revendications précédentes, incorporer en tant qu'unité compacte ayant un cadre permettant de supporter les multiples réacteurs à lit (2, 3) et un raccordement pour la ligne d'alimentation commune (1).

15. Système selon l'une quelconque des revendications précédentes, dans lequel tous les réacteurs à lit (2, 3) sont incorporés en tant que réacteur à lit fluidisé.

16. Utilisation d'un système de méthane selon l'une quelconque des revendications précédentes destiné à la synthèse de méthane.
